# EUROPEAN PATENT APPLICATION

(11) **EP 2 132 986 A1**
(43) Date of publication of application: **16.12.2009**
(21) Application number: 08738529.0
(22) Date of filing: 09.04.2008
(51) Int. Cl.: A01N 1/00, A61G 17/00

(54) **CONTAINER AND SUPPLY PIPE CONNECTING STRUCTURE, CONTAINER, AND SUPPLY PIPE**

(30) Priority: 09.04.2007 WO PCT/JP2007/057851
(71) Applicant: Nishihara, Risa, Hiroshima 730-0005 (JP)
(72) Inventor: Nishihara, Risa, Hiroshima 730-0005 (JP)
(74) Representative: Laufhütte, Dieter
(86) International application number: PCT/JP2008/000917
(87) International publication number: WO 2008/126412

(57) **Abstract**

A container for storing a body fluid leakage-preventing agent for a corpse includes a storage section of which the inside is filled with the body fluid leakage-preventing agent, a nozzle portion 13 provided in the storage section, and an engagement portion formed in the nozzle portion 13. A supply tube for injecting the body fluid leakage-preventing agent into the throat of the corpse includes an inserted tube portion 15 to be inserted toward the throat. An injection hole is formed on the distal end side of the inserted tube portion 15 in the insertion direction. A connecting portion 20 to be connected to the nozzle portion 13 of the container is provided on the proximal end side of the inserted tube portion 15. The connecting portion 20 is provided with an engagement portion to be engaged with an engagement portion of the nozzle portion 13.

## Description

### TECHNICAL FIELD

The present invention relates to a connection structure between a container and a supply tube, a container, and a supply tube for use in injecting a body fluid leakage-preventing agent into the throat of a corpse.

### BACKGROUND ART

Generally, a corpse, before being buried or cremated, needs to be preserved to suppress decay, and to prevent the generation of bad smell, discoloration, deformation, etc. Corpse preserving methods include, for example, a method in which a corpse is refrigerated or frozen with dry ice, and a method in which a preservative is applied on a corpse. Other methods include those in which a member with a preservative immersed therein, or a member supporting a preservative thereon, is placed on or around a corpse.

A body fluid may leak to the outside of a corpse from the throat through the nasal cavity or the oral cavity. As a method for suppressing the leakage of the body fluid, a method is known in the art (see, for example, Patent Document 1) in which a liquefied refrigeration gas is supplied into a corpse through the nasal cavity or the oral cavity to discharge the residue, etc., left in the body through the rectum. Also known in the art is a method in which a body fluid leakage-preventing agent is supplied into the throat (see, for example, Patent Document 2). A device used in the method of Patent Document 2 includes a container storing a body fluid leakage-preventing agent therein, and a supply tube to be inserted into the throat. A connecting portion to be connected to the container is provided at the proximal end portion of the supply tube. After the container is connected to the connecting portion of the supply tube, the supply tube is inserted into the throat, and the body fluid leakage-preventing agent in the container is injected into the throat via the supply tube so as to prevent the leakage of the body fluid.
Patent Document 1: Japanese Laid-Open Patent Publication No. 10-45501
Patent Document 2: Japanese Laid-Open Patent Publication No. 2002-275001

### DISCLOSURE OF THE INVENTION

### Problems To Be Solved By The Invention

While a liquefied refrigeration gas is injected into the nasal cavity or the oral cavity in the method disclosed in Patent Document 1, the liquefied refrigeration gas is not easily pushed into the corpse because the back of the nasal cavity or the oral cavity is often blocked by relaxed muscles. Particularly, where the liquefied refrigeration gas is injected into the oral cavity, the injection is very difficult because the back of the oral cavity is blocked by the tongue. Therefore, the method of Patent Document 1 may fail to completely prevent the leakage of the body fluid.

On the other hand, in the method disclosed in Patent Document 2, a supply tube is inserted into the throat of a corpse, with a body fluid leakage-preventing agent being injected by using the supply tube, and it is therefore possible to guide the body fluid leakage-preventing agent into the throat, in which regard the method is better than that of Patent Document 1. However, since the container and the supply tube are separate members, it is necessary to perform an operation of connecting these members together before the injection of the body fluid leakage-preventing agent. If the connection operation is not done securely, a body fluid leakage-preventing agent may possibly leak out from the connection portion between the container and the supply tube, or the supply tube may possibly be disconnected from the container during the injection operation, thus spraying around the body fluid leakage-preventing agent.

An object of the present invention is to ensure the connection between a container storing a body fluid leakage-preventing agent therein and a supply tube, and to prevent the body fluid leakage-preventing agent from leaking out from the connection portion, and to prevent the supply tube from being disconnected from the container.

### Means For Solving The Problems

In order to achieve the object set forth above, a first aspect is directed to a connection structure between a container and a supply tube, wherein the container stores a body fluid leakage-preventing agent for a corpse, and the supply tube supplies the body fluid leakage-preventing agent into a throat of the corpse, wherein the container includes a storage section of which an inside is filled with the body fluid leakage-preventing agent, a nozzle portion provided in the storage section, and an engagement portion provided in the nozzle portion, the supply tube includes an inserted tube portion to be inserted toward the throat of the corpse, an injection hole is formed on a distal end side of the inserted tube portion in an insertion direction, a connecting portion to be connected to the nozzle portion of the container is provided on a proximal end side of the inserted tube portion, and an engagement portion to be engaged with an engagement portion of the nozzle portion is provided in the connecting portion.

A second aspect is according to the first aspect, wherein the inserted tube portion and the connecting portion of the supply tube are molded together.

A third aspect is according to the first aspect, wherein the inserted tube portion and the connecting portion of the supply tube are separately molded each by using a resin material, and then welded together into an integral piece.

A fourth aspect is according to one of the first to third aspects, wherein an outer periphery surface of the nozzle portion of the container is formed by a tapered surface with its diameter decreasing toward a distal end of the nozzle portion, the connecting portion of the supply tube has a cylindrical shape such as to surround the tapered surface of the nozzle portion, and an inner periphery surface of the connecting portion is formed so as to conform with the tapered surface.

A fifth aspect is according to the fourth aspect, wherein the engagement portion of the nozzle portion is provided on the tapered surface, and the engagement portion of the connecting portion is provided on the inner periphery surface of the connecting portion.

A sixth aspect is according to the fourth or fifth aspect, wherein one of the engagement portion of the nozzle portion and the engagement portion of the connecting portion is a projecting portion, and the other is a depressed portion that fits around the projecting portion.

A seventh aspect is according to the fourth or fifth aspect, wherein a through hole is formed in a peripheral wall portion of one of the nozzle portion and the connecting portion, and a protruding portion to be inserted into the through hole is formed on a peripheral wall portion of the other, the through hole and the protruding portion each being the engagement portion.

An eighth aspect is according to the sixth aspect, wherein the depressed portion is a groove portion, and the projecting portion is a protruding portion to be inserted into the groove portion.

A ninth aspect is according to the fourth or fifth aspect, wherein a peripheral wall portion of one of the nozzle portion and the connecting portion is provided with a first groove portion extending in a circumferential direction in an axially intermediate portion thereof, and a second groove portion continuously extending with the first groove portion and extending in an axial direction along the peripheral wall portion, and a peripheral wall portion of the other is provided with a pin portion to be inserted in the first groove portion and the second groove portion.

A tenth aspect is according to the fourth or fifth aspect, wherein a peripheral wall portion of one of the nozzle portion and the connecting portion is provided with a first depressed portion extending in a circumferential direction in an axially intermediate portion thereof, and a second depressed portion continuously extending with the first depressed portion and extending in an axial direction along the peripheral wall portion, and a peripheral wall portion of the other is provided with a pin portion to be inserted in the first depressed portion and the second depressed portion.

An eleventh aspect is according to one of the first to tenth aspects, wherein a cap for closing a distal end opening of the nozzle portion of the container is engaged with the engagement portion of the nozzle portion.

A twelfth aspect is according to one of the first to eleventh aspects, wherein the storage section of the container is a cylindrical portion of a syringe, and a piston for pushing out the body fluid leakage-preventing agent is inserted into the cylindrical portion of the syringe.

A thirteenth aspect is according to one of the first to twelfth aspects, wherein the distal end side of the inserted tube portion of the supply tube is curved.

A fourteenth aspect is according to one of the first to thirteenth aspects, wherein a tapered portion whose diameter decreases toward a distal end may be provided on the distal end side of the inserted tube portion of the supply tube.

A fifteenth aspect is according to one of the first to fourteenth aspects, wherein a thickness of the inserted tube portion of the supply tube is set to be smaller than a thickness of the connecting portion.

A sixteenth aspect is according to one of the first to fifteenth aspects, wherein the supply tube is provided with a mark indicating an amount of insertion of the inserted tube portion into the throat.

A seventeenth aspect is according to one of the first to fifteenth aspects, wherein the supply tube is provided with a stopper that abuts on a portion of the corpse when the injection hole of the inserted tube portion inserted toward the throat reaches a predetermined position.

An eighteenth aspect is according to one of the first to fifteenth aspects, wherein the supply tube is provided with a mark indicating an amount of insertion and/or a stopper for controlling the amount of insertion to be a predetermined amount when the inserted tube portion is inserted from a nasal cavity toward the throat, and a mark indicating an amount of insertion and/or a stopper for controlling the amount of insertion to be a predetermined amount when the inserted tube portion is inserted from an oral cavity toward the throat.

A nineteenth aspect is according to one of the first to fifteenth aspects, wherein the supply tube is provided with a marking member indicating an amount of insertion of the inserted tube portion into the throat.

A twentieth aspect is according to one of the first to fifteenth aspects, wherein the connection structure includes: a first inserted tube portion to be inserted from a nasal cavity toward the throat; and a second inserted tube portion to be inserted from an oral cavity toward the throat.

A twenty-first aspect is according to one of the first to twentieth aspects, wherein a plurality of the injection holes of the supply tube are formed in a peripheral wall portion of the inserted tube portion on the distal end side thereof.

A twenty-second aspect is directed to a container to which a supply tube for supplying a body fluid leakage-preventing agent into a throat of a corpse is connected, the container including: a storage section filled with the body fluid leakage-preventing agent; a nozzle portion provided in the storage section; and an engagement portion provided in the nozzle portion, the nozzle portion being connected to a connecting portion provided in the supply tube, wherein the engagement portion of the nozzle portion is engaged with an engagement portion of the connecting portion.

A twenty-third aspect is directed to a supply tube connected to a container storing a body fluid leakage-preventing agent for a corpse, including an inserted tube portion to be inserted toward a throat of the corpse, wherein an injection hole is formed on a distal end side of the inserted tube portion in an insertion direction, a connecting portion to be connected to a nozzle portion provided in the container is provided on a proximal end side of the inserted tube portion, and the connecting portion is provided with an engagement portion to be engaged with an engagement portion of the nozzle portion.

"Throat" as used herein refers to what is commonly called "the throat", and includes the pharynx, the larynx, and the vicinity thereof.

Before the body fluid leakage-preventing agent is injected from the injection hole of the inserted tube portion of the supply tube into the throat, the distal end portion of the inserted tube portion may reach the throat or may reach deeper than the throat. If the distal end portion of the inserted tube portion has reached deep in the throat, an injection hole can be opened at a position closer to the proximal end than the distal end portion of the inserted tube portion so that this injection hole is positioned in the throat.

The inserted tube portion of the supply tube may be inserted to a position before the throat, so that the body fluid leakage-preventing agent discharged from the injection hole of the inserted tube portion is allowed to flow into the throat.

The body fluid leakage-preventing agent may be an agent disclosed in Japanese Laid-Open Patent Publication No. 2002-275001, e.g., a body fluid leakage-preventing agent in which a superabsorbent polymer powder capable of absorbing the body fluid is dispersed in a viscous fluid. A body fluid leakage-preventing agent in which the superabsorbent polymer powder is dispersed in a cream-like base agent or a mousse-like base agent may also be used.

The body fluid leakage-preventing agent may also be one that is obtained by wrapping the superabsorbent polymer powder in a deformable bag. In such a case, the superabsorbent polymer wrapped in the bag is pushed out from the container through the supply tube.

### Effects Of The Invention

According to the first aspect, the container and the supply tube can be connected together by engaging the engagement portion provided in the connecting portion of the supply tube with the engagement portion of the nozzle portion of the container. Thus, by engaging the engagement portion of the container with the engagement portion of the supply tube, it is possible to ensure the connection between the container and the supply tube. Therefore, during the operation of injecting the body fluid leakage-preventing agent into a corpse, it is possible to prevent the body fluid leakage-preventing agent from leaking out from the connection portion, and to prevent the supply tube from coming off the container.

According to the second aspect, molding together the inserted tube portion and the connecting portion of the supply tube eliminates the need for an adhesive, which is used when they are molded separately. Thus, no adhesive will run over from between the inserted tube portion and the connecting portion into the inner passage of the inserted tube portion, thereby preventing the inner passage from being closed by an adhesive.

According to the third aspect, the inserted tube portion and the connecting portion of the supply tube can be made into an integral piece without using an adhesive, thereby avoiding the problems associated with the use of an adhesive. The hardness and/or the material can be varied between the inserted tube portion and the connecting portion, thus improving the freedom in designing the supply tube.

According to the fourth aspect, with the container and the supply tube connected together, the inner periphery surface of the connecting portion of the supply tube is fit around the tapered surface of the nozzle portion. Thus, the tapered surface of the nozzle portion and the inner periphery surface of the connecting portion can be brought into close contact with each other. Therefore, it is possible to increase the liquid impermeability.

According to the fifth aspect, with the inner periphery surface of the connecting portion of the supply tube fit around the tapered surface of the nozzle portion, the engagement portions are engaged with each other. Thus, it is possible to better prevent the supply tube from coming off the container.

According to the sixth to eighth aspects, the engagement portions can be engaged with each other in a simple operation.

According to the ninth aspect, when the nozzle portion is inserted into the connecting portion, the pin portion is inserted into the second groove portion, and when the pin portion reaches the proximal end of the second groove portion, the nozzle portion is rotated around the axial line thereof with respect to the connecting portion, thus inserting the pin portion into the first groove portion. Thus, the pin portion and the first groove portion are engaged with each other, making it unlikely that the connecting portion comes off the nozzle portion. By the rotation operation, the tapered surface of the nozzle portion and the inner periphery surface of the connecting portion can be brought more strongly into close contact with each other. Thus, it is possible to improve the liquid impermeability between the nozzle portion and the connecting portion. Moreover, the supply tube can be pulled off the container by performing an operation opposite to the operation described above, thereby improving the operability. Note that when the nozzle portion is connected to the connecting portion, the connecting portion may be rotated with respect to the nozzle portion.

According to the tenth aspect, as in the ninth aspect, the pin portion and the first depressed portion are engaged with each other, thereby making it unlikely that the connecting portion comes off the nozzle portion, and improving the liquid impermeability between the nozzle portion and the connecting portion, and it is also possible to improve the operability in pulling the supply tube off the container.

According to the eleventh aspect, since the cap for closing the distal end opening of the nozzle portion is engaged with the engagement portion of the nozzle portion, it is possible to prevent the body fluid leakage-preventing agent in the container from inadvertently leaking out before use.

According to the twelfth aspect, the body fluid leakage-preventing agent can be reliably held in the syringe before use, and the body fluid leakage-preventing agent can be reliably injected into the throat by a simple operation of pushing the piston into the syringe.

According to the thirteenth aspect, since the distal end side of the inserted tube portion of the supply tube is curved, when the inserted tube portion is inserted toward the throat from the nasal cavity or the oral cavity of a corpse, for example, it can be smoothly inserted so as to conform with the curved shape of the area into the throat of the corpse. Thus, the operation of inserting the inserted tube portion can be performed with a light force and quickly.

According to the fourteenth aspect, since the tapered portion is provided on the distal end side of the inserted tube portion of the supply tube, when the inserted tube portion of the supply tube is inserted into the throat from the nasal cavity or the oral cavity, for example, the distal end portion of the inserted tube portion is less likely to be stuck on the inner wall of the nasal cavity or the oral cavity. Thus, the inserted tube portion can be smoothly inserted into the throat.

According to the fifteenth aspect, it is possible to make the inserted tube portion soft, while sufficiently ensuring a rigidity of the connecting portion of the supply tube to thereby ensure the connection between the container and the nozzle portion. By making the inserted tube portion soft, the inserted tube portion, when being inserted, can easily deform so as to conform with the curved shape of the area into the throat of the corpse, thus improving the insertion operability.

According to the sixteenth aspect, when the inserted tube portion of the supply tube is inserted toward the throat, it is possible to easily determine when to stop the insertion operation. Thus, the inserted tube portion can be reliably inserted into the throat without requiring skills of the operator.

According to the seventeenth aspect, since the stopper is provided on the supply tube, when the inserted tube portion is inserted toward the throat, the operator can insert the inserted tube portion to a predetermined position by stopping the insertion operation at, or slightly before, the point where the stopper contacts the corpse. Thus, the inserted tube portion can be reliably inserted into the throat to inject the body fluid leakage-preventing agent therethrough without requiring skills of the operator.

According to the eighteenth aspect, a single supply tube can be used both for the insertion into the throat through the nasal cavity and for the insertion into the throat through the oral cavity, thereby improving the versatility.

According to the nineteenth aspect, the supply tube can be shared between when the marking member is used and when it is not used.

According to the twentieth aspect, since different supply tubes suitable for the nasal cavity use and the oral cavity use are provided, the body fluid leakage-preventing agent can be injected either way as necessary.

According to the twenty-first aspect, when some of the injection holes of the inserted tube portion are blocked by the mucous membrane in the throat, or the like, the body fluid leakage-preventing agent can be reliably injected into the throat through other injection holes.

According to the twenty-second and twenty-third aspects, by engaging the engagement portion of the container and the engagement portion of the supply tube with each other, it is possible to ensure the connection between the container and the supply tube. Thus, it is possible to prevent the body fluid leakage-preventing agent from leaking out from the connection portion, and to prevent the supply tube from coming off the container.

By injecting the body fluid leakage-preventing agent into the throat and keeping it in the throat as described above, it is possible to prevent the body fluid leakage.

### BRIEF DESCRIPTION OF THE DRAWINGS

[FIG. 1] A cross-sectional view showing a supply tube according to Embodiment 1.
[FIG. 2] A side view showing a supply tube according to Embodiment 1.
[FIG. 3] A cross-sectional view showing a container according to Embodiment 1.
[FIG. 4] A side view showing a container according to Embodiment 1.
[FIG. 5] An enlarged cross-sectional view showing a connection portion between a container and a supply tube.
[FIG. 6] An enlarged view showing the inside of W in FIG. 5.
[FIG. 7] A diagram illustrating an embalming device being used, to which a connection structure between a container and a supply tube according to Embodiment 1 is applied.
[FIG. 8] A view equivalent to FIG. 6 for a variation of Embodiment 1.
[FIG. 9] A perspective view showing a supply tube according to Embodiment 2.
[FIG. 10] A view equivalent to FIG. 1 for Embodiment 2.
[FIG. 11] A side view showing a connecting portion and a nozzle portion according to Variation 1 of Embodiment 2.
[FIG. 12] A view equivalent to FIG. 11 for Variation 2 of Embodiment 2.
[FIG. 13] A cross-sectional view for Variation 3 of Embodiment 2, showing a state where a cap is put on a nozzle portion.
[FIG. 14] A view equivalent to FIG. 13 for Variation 4 of Embodiment 2.
[FIG. 15] A view equivalent to FIG. 1 for Variation 5 of Embodiment 2.
[FIG. 16] A cross-sectional view showing a proximal end side of a supply tube according to Variation 6 of Embodiment 2.
[FIG. 17] A view equivalent to FIG. 16 for Variation 7 of Embodiment 2.
[FIG. 18] A cross-sectional view showing a nozzle portion according to Variation 7 of Embodiment 2.
[FIG. 19] A cross-sectional view showing, on an enlarged scale, a connection portion between a container and a supply tube according to Variation 7 of Embodiment 2.
[FIG. 20] A cross-sectional view showing a distal end side of a supply tube according to Variation 8 of Embodiment 2.
[FIG. 21] A view equivalent to FIG. 20 for Variation 9 of Embodiment 2.
[FIG. 22] A view equivalent to FIG. 20 for Variation 10 of Embodiment 2.
[FIG. 23] A view equivalent to FIG. 7 for Embodiment 3.
[FIG. 24] A view equivalent to FIG. 9 for Embodiment 3.
[FIG. 25] A view equivalent to FIG. 9 for Variation 1 of Embodiment 3.
[FIG. 26] A view equivalent to FIG. 9 for Variation 2 of Embodiment 3.
[FIG. 27] A view equivalent to FIG. 24 for Embodiment 4.

### DESCRIPTION OF REFERENCE NUMERALS

- 1: Embalming device
- 2: Body fluid leakage-preventing agent
- 3: Container
- 4, 31: Supply tube
- 10: Syringe
- 11: Piston
- 12: Storage section
- 13: Nozzle portion
- 13a: Opening
- 14: Protruding portion (engagement portion)
- 15: Inserted tube portion
- 16 to 18: First to third injection holes
- 20, 34: Connecting portion
- 22: Stopper (mark)
- 23: Groove portion (engagement portion)
- 25: Cap
- 30: Through hole (engagement portion)
- 35: Through hole (engagement portion)
- 38: Stopper
- 40: First groove portion
- 41: Second groove portion
- 42: Pin portion

### BEST MODE FOR CARRYING OUT THE INVENTION

Preferred embodiments of the present invention will now be described with reference to the drawings. Note that the following description of preferred embodiments is merely illustrative of the present invention, and is not intended to limit the present invention itself or the application thereof.

### First Embodiment

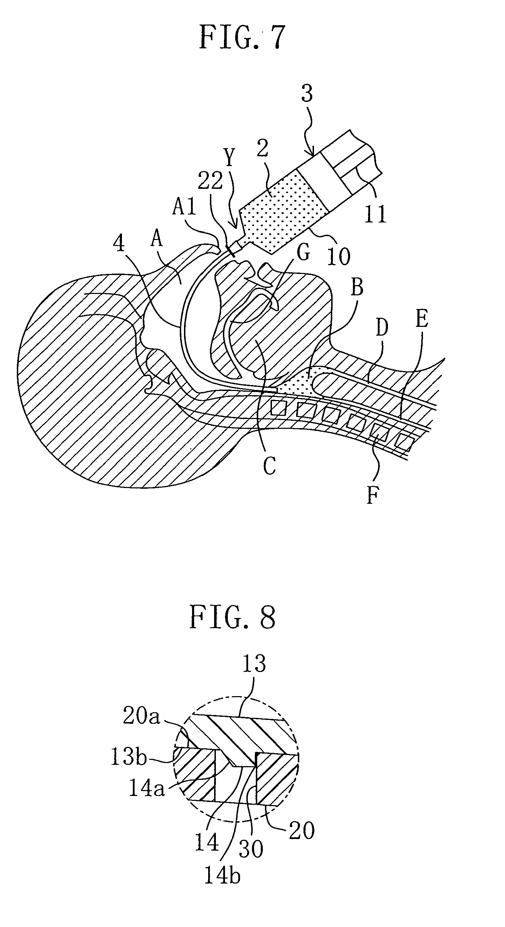

FIG. 7 is a diagram illustrating an embalming device 1 being used, in which a connection structure Y between a container and a supply tube according to Embodiment 1 of the present invention is employed.

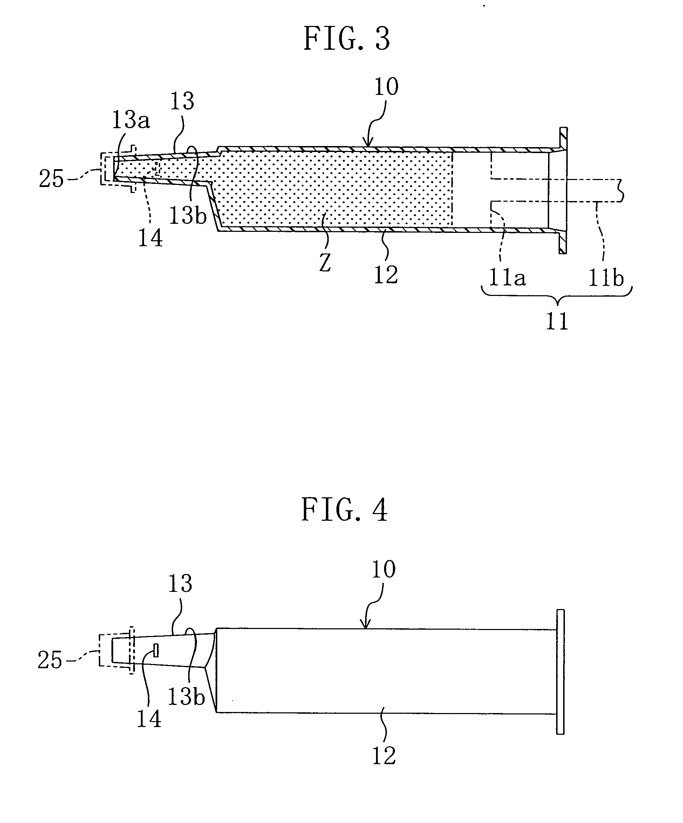

The embalming device 1 includes a container 3 for storing a body fluid leakage-preventing agent 2 for a corpse, and a supply tube 4 for supplying the body fluid leakage-preventing agent 2 into the throat B of a corpse. The container 3 includes a syringe 10 and a piston 11. The syringe 10 includes a cylindrical storage section 12 of which the inside is filled with the body fluid leakage-preventing agent 2, and a nozzle portion 13 provided in the storage section 12, as shown in FIG. 3.

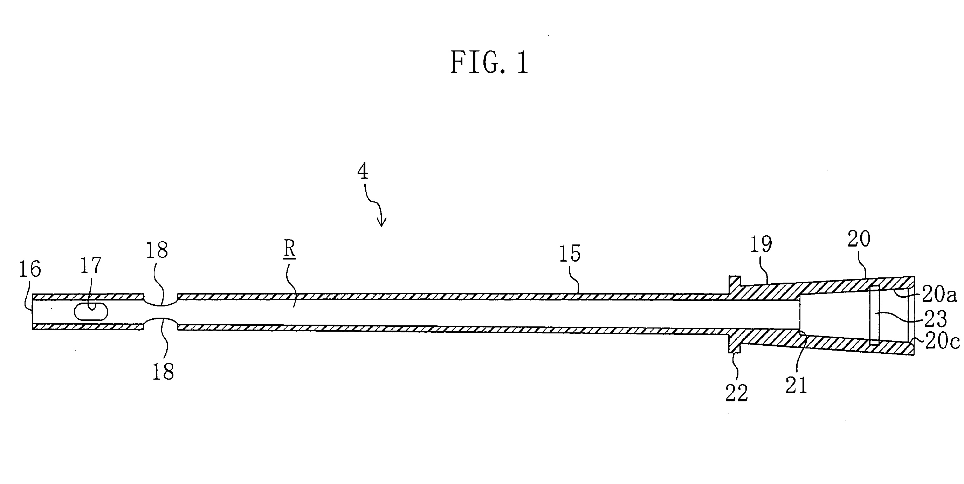

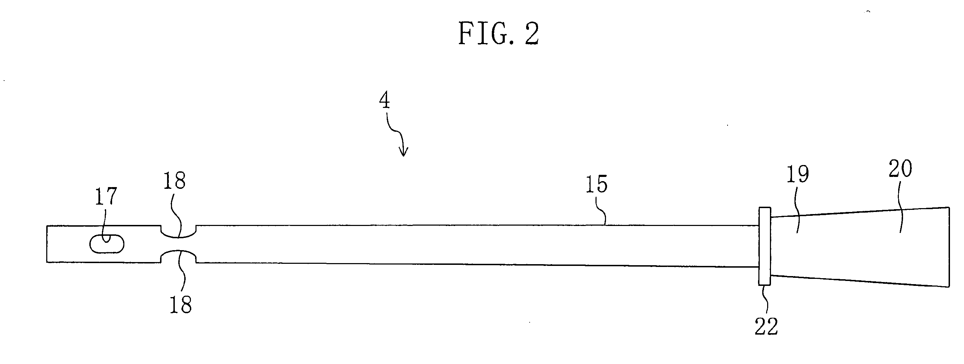

The supply tube 4 includes an inserted tube portion 15 to be inserted from the nasal cavity A toward the throat B, as shown in FIGS. 1 and 2. The inserted tube portion 15 has a circular cross section. First, second and third injection holes 16, 17 and 18 are formed on the distal end side (the left side in FIGS. 1 and 2) of the inserted tube portion 15 in the insertion direction. A cylindrical intermediate portion 19 is provided on the proximal end portion (the right side in FIGS. 1 and 2) of the inserted tube portion 15. A connecting portion 20 to be connected to the nozzle portion 13 of the container 3 is provided in a proximal end portion of the intermediate portion 19. The inserted tube portion 15, the intermediate portion 19 and the connecting portion 20 are molded together by using a flexible synthetic resin such as vinyl chloride.

The inserted tube portion 15 is in an elongated shape. The inserted tube portion 15 is so flexible that it can be deformed in conformity with the inner shape from the nasal cavity A to the throat B and is so rigid that an inner passage R of the inserted tube portion 15 will not collapse when inserted into the nasal cavity A. The first injection hole 16 is opened at the distal end surface of the inserted tube portion 15 in the insertion direction, as shown in FIG. 1. Two of the second injection holes 17 and 17 are opened, 180° apart from each other in the circumferential direction, in a peripheral wall portion of the inserted tube portion 15 that is away from the distal end surface thereof toward the proximal end side. Two of the third injection holes 18 and 18 are opened, 180° apart from each other, in a peripheral wall portion of the inserted tube portion 15 that is further away from the second injection holes 17 and 17 toward the proximal end side. The opening positions of the second injection holes 17 and 17 and the opening positions of the third injection holes 18 and 18 are shifted by 90° from each other in the circumferential direction of the inserted tube portion 15. The first injection hole 16 is in a circular shape, and the second and third injection holes 17 and 18 are in a generally elliptical shape.

The longer axis dimension of the second and third injection holes 17 and 18 is set to 4 mm, and the shorter axis dimension thereof is set to 3 mm. The centers of the second injection holes 17 are 10 mm away from the distal end surface of the inserted tube portion 15 toward the proximal end side. The centers of the third injection holes 18 are 22 mm away from the distal end surface of the inserted tube portion 15 toward the proximal end side.

The first to third injection holes 16 to 18 are not limited to the embodiment above as long as the body fluid leakage-preventing agent 2 in the inserted tube portion 15 can be injected therethrough into the throat B. That is, the shape, the number and the opening positions of the first to third injection holes 16 to 18 can be determined arbitrarily, depending on various conditions such as the outer diameter and the inner diameter of the inserted tube portion 15, the elasticity of the inserted tube portion 15, and the type and the fluidity of the body fluid leakage-preventing agent 2 flowing through the inner passage R. For example, the opening areas of the second and third injection holes 17 and 18 may be different from each other. The second and third injection holes 17 and 18 may be in a circular shape. Moreover, one or two of the first to third injection holes 16 to 18 may be omitted. Although not shown, another injection hole may be opened further toward proximal end side than the third injection holes 18 of the inserted tube portion 15.

The spacing between the centers of the second injection holes 17 and the distal end surface of the inserted tube portion 15 is preferably 6 mm or more and 15 mm or less. The centers of the second injection holes 17 and the centers of the third injection holes 18 are preferably spaced apart from each other in the axial direction of the inserted tube portion 15 by a distance in the range of 4 mm or more and 10 mm or less. By determining the sizes and the positions of the second and third injection holes 17 and 18 as described above, the body fluid leakage-preventing agent 2 in the inserted tube portion 15 can be reliably injected into the throat B.

The outer diameter of the inserted tube portion 15 on the distal end side is set to 5.0 mm, and the inner diameter thereof is set to 3.5 mm. The outer diameter and the inner diameter of the inserted tube portion 15 gradually increase from the distal end portion toward the proximal end portion. This is to take into consideration the mold releasability in the mold process of the inserted tube portion 15.

The outer diameter of the inserted tube portion 15 is preferably 2.5 mm or more and 8.0 mm or less. The inner diameter of the inserted tube portion 15 is preferably set to be smaller than the outer diameter by a length in the range of 0.6 mm or more and 2.4 mm or less. The upper limit of the outer diameter of the inserted tube portion 15 is set to 8.0 mm or less because the inserted tube portion 15 will not be easily inserted into the nasal cavity A if the outer diameter is greater than 8.0 mm. The lower limit of the outer diameter of the inserted tube portion 15 is set to 2.5 mm or less because the inner passage R will be narrowed to inhibit the flow of the body fluid leakage-preventing agent 2 therethrough if the outer diameter is smaller than 2.5 mm. That is, by setting the outer diameter and the inner diameter of the inserted tube portion 15 as described above, it is possible to improve the operability in inserting the inserted tube portion 15 through the nasal cavity A and the operability in injecting the body fluid leakage-preventing agent 2 therethrough.

The inner diameter of the intermediate portion 19 is set to be equal to the inner diameter of the proximal end portion of the inserted tube portion 15. The inner periphery surface of the intermediate portion 19 smoothly connects to the inner periphery surface of the inserted tube portion 15. The outer diameter of the intermediate portion 19 is set to be greater than the outer diameter of the inserted tube portion 15, and gradually increases toward the connecting portion 20. The outer periphery surface of the intermediate portion 19 smoothly connects to the outer periphery surface of the connecting portion 20. The thickness of the intermediate portion 19 is set to be greater than the thickness of the inserted tube portion 15.

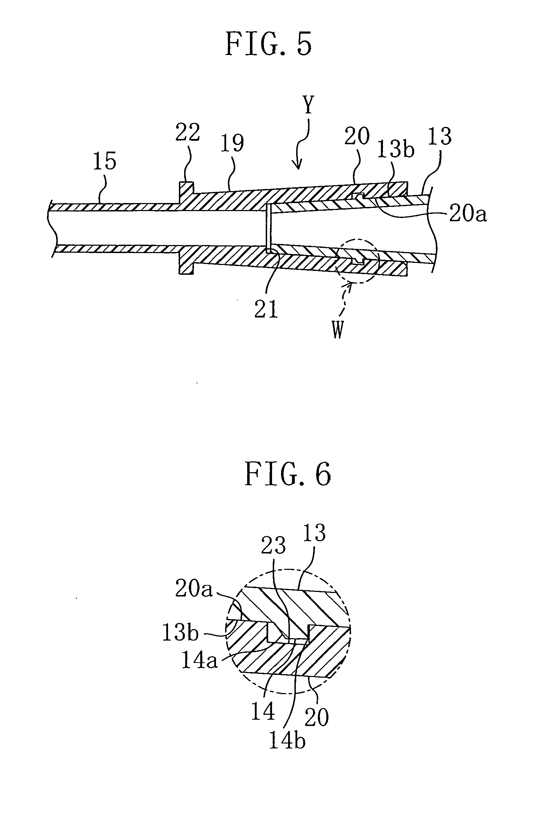

The connecting portion 20 has a cylindrical shape such as to surround the nozzle portion 13 of the container 3. The distal end portion of the connecting portion 20 (the side on which it continuously extends to the intermediate portion 19) is molded together with the proximal end portion of the intermediate portion 19. The inner diameter of the connecting portion 20 is set to be greater than the inner diameter of the intermediate portion 19, and specifically corresponds to the outer diameter of the nozzle portion 13. Therefore, a step portion 21 is formed between the inner periphery surface of the intermediate portion 19 and the inner periphery surface of the connecting portion 20, and the thickness of the connecting portion 20 is smaller than the thickness of the intermediate portion 19. The thickness of the connecting portion 20 is greater than the thickness of the inserted tube portion 15. The depth of the step portion 21 is set to be generally equal to the thickness of the nozzle portion 13, as shown in FIG. 5, so that the inner periphery surface of the nozzle portion 13 continuously extends with the inner periphery surface of the intermediate portion 19, with the nozzle portion 13 inserted in the connecting portion 20.

The inner periphery surface of the connecting portion 20 is formed so as to flare toward the proximal end thereof, and is formed by an engagement surface 20a to be engaged with the outer periphery surface of the nozzle portion 13. A groove portion (an engagement portion) 23 extending in the circumferential direction is provided in an axially intermediate portion of the engagement surface 20a. The groove portion 23 continuously extends in a ring shape around the entire circumference of the engagement surface 20a.

A chamfered portion 20c, which is chamfered, is formed on the inner periphery surface of the proximal end portion of the connecting portion 20. Due to the chamfered portion 20c, the inner diameter of the proximal end portion of the connecting portion 20 is increased. This enables the nozzle portion 13 of the syringe 10 to be easily inserted into the connecting portion 20.

A stopper 22 is formed together with the inserted tube portion 15, the stopper 22 extending in the circumferential direction while protruding in the radially outward direction from the vicinity of the boundary between the inserted tube portion 15 and the intermediate portion 19. The stopper 22 is positioned so that the stopper 22 abuts on the nose tip A1 when the first to third injection holes 16 to 18 of the inserted tube portion 15 reach the throat B. Note that the position of the stopper 22 may be determined so that the stopper 22 abuts on the nose tip A1 when the distal end portion of the inserted tube portion 15 is located in the vicinity of the throat B. The shape of the stopper 22 is not limited to that described above, but may be a shape that extends interruptedly in the circumferential direction of the inserted tube portion 15, or a projecting shape that is protruding from a portion along the circumference of the inserted tube portion 15. The stopper 22 may be formed at another position of the inserted tube portion 15 on the proximal end side, or on the intermediate portion 19 or the connecting portion 20.

Instead of the stopper 22, a mark (marking) indicating the amount of insertion of the inserted tube portion 15 into the throat B may be provided. The position of the mark may be determined as with the stopper 22. The stopper 22 may be used as a mark. A plurality of marks may be provided at intervals in the axial direction of the inserted tube portion 15. A mark may be a projecting portion, a depressed portion, or the like, or may be letters, a line, or the like.

The syringe 10 is an integral molded piece of a resin material. As shown in FIGS. 3 and 4, the nozzle portion 13 extends along a straight line, with the axial line thereof being shifted from the axial line of the syringe 10. An opening 13a, through which the body fluid leakage-preventing agent 2 is discharged, is formed at the tip of the nozzle portion 13. The outer periphery surface of the nozzle portion 13 is formed by a tapered surface 13b with the diameter decreasing toward the distal end side, and the shape of the tapered surface 13b is determined so that it extends to conform with the engagement surface 20a of the connecting portion 20 of the supply tube 4 and so that it fits to the engagement surface 20a. Therefore, as the nozzle portion 13 is inserted in the connecting portion 20 of the supply tube 4, the engagement surface 20a and the tapered surface 13b are in close contact with each other along the entire circumference, thus ensuring a sufficient liquid impermeability.

Two protruding portions (engagement portions) 14 and 14 are formed, spaced apart from each other in the circumferential direction, on the outer periphery surface of the axially intermediate portion of the nozzle portion 13. As shown in FIG. 6, the protruding portions 14 and 14 are arranged so as to be positioned in the groove portion 23, with the nozzle portion 13 completely inserted in the connecting portion 20 of the supply tube 4, and are formed so as to fit in the groove portion 23.

Each protruding portion 14 includes, on the distal end side thereof in the insertion direction, a slant portion 14a, which is inclined toward the proximal end side. On the other hand, an edge portion 14b is formed on the proximal end side of the protruding portion 14.

Note that the number of the protruding portions 14 of the nozzle portion 13 is not limited to two, but may be one or three or more. The protruding portion 14 may be formed in a ring shape that is continuous in the circumferential direction of the nozzle portion 13.

As shown in FIGS. 3 and 4, a resin cap 25 for closing the opening 13a is put on the nozzle portion 13. Therefore, the body fluid leakage-preventing agent 2 in the storage section 12 will not leak out from the nozzle portion 13 before use.

The piston 11 is for pushing the body fluid leakage-preventing agent 2 inside the storage section 12 out from the opening 13a of the nozzle portion 13. The piston 11 includes a pushing portion 11a to be inserted from an end portion of the storage section 12 that is opposite to the nozzle portion 13, and a pushing rod 11b for operating the pushing portion 11a. The pushing portion 11a and the pushing rod 11b are formed by a resin material similar to that of the syringe 10. Therefore, after use, the syringe 10 and the piston 11 can be disposed of together.

Next, the method for connecting together the container 3 and the supply tube 4 of the embalming device 1 having such configurations will be described. Note that the cap 25 is put on the nozzle portion 13 of the syringe 10, which is filled with the body fluid leakage-preventing agent 2. The pushing portion 11 a of the piston 11 is inserted in the syringe 10, thereby closing the syringe 10.

First, the cap 25 is taken off the nozzle portion 13. Then, the container 3 and the supply tube 4 are connected together. That is, the nozzle portion 13 of the syringe 10 is inserted into the connecting portion 20 of the supply tube 4. As the nozzle portion 13 is inserted deeper into the connecting portion 20, the tapered surface 13b of the nozzle portion 13 is pushed against the engagement surface 20a of the connecting portion 20. At this point, the tapered surface 13b and the engagement surface 20a are brought into close contact with each other, with a slight elastic deformation, since the nozzle portion 13 and the connecting portion 20 are formed by a resin material. At a point immediately before the nozzle portion 13 is completely inserted in the connecting portion 20, the protruding portions 14 of the nozzle portion 13 start fitting into the groove portion 23 of the connecting portion 20. At this point, since the slant portion 14a is formed on the distal end side of each protruding portion 14 in the insertion direction, the protruding portions 14 easily fit into the groove portion 23. Then, the protruding portions 14 are engaged with the groove portion 23 when the protruding portions 14 are completely fit in the groove portion 23, i.e., when the nozzle portion 13 is completely inserted in the connecting portion 20, as shown in FIG. 5. This prevents the nozzle portion 13 from inadvertently coming off the connecting portion 20, and keeps a close contact between the tapered surface 13b of the nozzle portion 13 and the engagement surface 20a of the connecting portion 20, thereby ensuring a high liquid impermeability.

If a force acts upon the nozzle portion 13 in such a direction that urges the nozzle portion 13 to come off the connecting portion 20, with the nozzle portion 13 inserted in the connecting portion 20, the edge portions 14b of the protruding portions 14 are pushed into the inner surface of the groove portion 23. This prevents the nozzle portion 13 from coming off.

Since the thickness of the connecting portion 20 of the supply tube 4 is greater than the thickness of the inserted tube portion 15, as described above, the rigidity of the connecting portion 20 is higher than that of the inserted tube portion 15. Thus, even if an external force acts upon the connecting portion 20, it is possible to keep a close contact between the connecting portion 20 and the nozzle portion 13.

Next, the method for injecting the body fluid leakage-preventing agent 2 into the throat B will be described. Note that in FIG. 7, C denotes the tongue, D denotes the airway, E denotes the esophagus, F denotes the cervical vertebra, and G denotes the oral cavity.

First, the inserted tube portion 15 is inserted into the nasal cavity A from the distal end side of the inserted tube portion 15. At this point, since the inserted tube portion 15 is thinner and more flexible than the intermediate portion 19 or the connecting portion 20, the inserted tube portion 15 can easily deform to conform with the inner shape of the nasal cavity A, thus requiring only a small insertion force.

As the inserted tube portion 15 is inserted deeper into the nasal cavity A, and the first to third injection holes 16 to 18 of the inserted tube portion 15 reach the throat B, the stopper 22 abuts on the nose tip A1. That is, the stopper 22 serves as a marking indicating that the distal end side of the inserted tube portion 15 has reached the throat B. Once the stopper 22 abuts on the nose tip A1, the inserted tube portion 15 cannot be inserted any further.

On the other hand, if the operator stops inserting the inserted tube portion 15, with the stopper 22 being substantially away from the nose tip A1, the distal end side of the inserted tube portion 15 has not reached the throat B. If it has not reached the throat B, the inserted tube portion 15 is pushed further into the nasal cavity A.

If there is a strong insertion resistance when pushing the inserted tube portion 15 into the nasal cavity A, the inserted tube portion 15 is once pulled back and then re-inserted, or is re-inserted through the other nasal cavity A. In this way, the inserted tube portion 15 can be inserted toward the throat B. Therefore, even a person with little embalming experience can easily insert the inserted tube portion 15 into the throat B.

Then, as the pushing rod 11b of the piston 11 is pushed into the syringe 10, the pushing portion 11a slides against the inner surface of the syringe 10 toward the nozzle portion 13. Thus, the body fluid leakage-preventing agent 2 in the syringe 10 is passed through the nozzle portion 13, the opening 13a and the intermediate portion 19 of the supply tube 4 into the inserted tube portion 15. The body fluid leakage-preventing agent 2, which has been passed into the inserted tube portion 15, passes through the inner passage R and is injected into the throat B through the first to third injection holes 16 to 18. During this injection of the body fluid leakage-preventing agent 2, the pressure from the pushing portion 11a acts on the area from the inside of the nozzle portion 13 to the inside of the supply tube 4. Nevertheless, since the tapered surface 13b and the engagement surface 20a are in close contact with each other and since the protruding portions 14 are fit in the groove portion 23, as described above, the nozzle portion 13 is prevented from inadvertently coming off the connecting portion 20, and the body fluid leakage-preventing agent 2 is prevented from leaking out from between the nozzle portion 13 and the connecting portion 20.

When the body fluid leakage-preventing agent 2 is injected into the throat B, part or whole of the second injection holes 17 of the inserted tube portion 15, for example, may possibly be blocked by the inner wall of the nasal cavity A or the inner wall of the throat B. In such a case, the body fluid leakage-preventing agent 2 is discharged from the third injection holes 18 so as to be guided to the throat B through between the outer periphery surface of the inserted tube portion 15 and the inner wall of the nasal cavity A, and is injected into the throat B from the first injection hole 16.

Other than the method in which the inserted tube portion 15 of the supply tube 4 is inserted into the nasal cavity A after the container 3 and the supply tube 4 are connected together as described above, the method for injecting the body fluid leakage-preventing agent 2 into the throat B may be a method in which the container 3 and the supply tube 4 are connected together after the inserted tube portion 15 of the supply tube 4 is inserted into the nasal cavity A.

In the latter method, first, the inserted tube portion 15 is inserted from the nasal cavity A toward the throat B. Then, the nozzle portion 13 of the syringe 10 is inserted into the connecting portion 20 of the supply tube 4 so that the protruding portions 14 are engaged with the groove portion 23. Thus, the container 3 and the supply tube 4 are connected together. Then, by operating the piston 11, the body fluid leakage-preventing agent 2 is injected into the throat B via the supply tube 4.

Therefore, in the connection structure Y between the container 3 and the supply tube 4 according to Embodiment 1, the protruding portions 14 are formed on the outer periphery surface of the nozzle portion 13 of the container 3 and the groove portion 23 is formed on the inner periphery surface of the connecting portion 20 of the supply tube 4, so that the protruding portions 14 are fit in the groove portion 23 with the nozzle portion 13 inserted in the connecting portion 20, thus ensuring the connection between the container 3 and the supply tube 4. This prevents the body fluid leakage-preventing agent 2 from leaking out from the connection portion during the operation of injecting the body fluid leakage-preventing agent 2 into a corpse, and prevents the supply tube 4 from coming off the container 3.

Molding together the inserted tube portion 15 and the connecting portion 20 of the supply tube 4 eliminates the need for an adhesive, which is used when they are molded separately. Thus, no adhesive will run over from between the inserted tube portion 15 and the connecting portion 20 into the inner passage R of the inserted tube portion 15, thereby preventing the inner passage R from being closed by an adhesive.

Since the protruding portions 14 fit in the groove portion 23, with the engagement surface 20a of the connecting portion 20 of the supply tube 4 fit over the tapered surface 13b of the nozzle portion 13, the protruding portions 14 are unlikely to be detached from the groove portion 23. Thus, it is possible to even more reliably connect together the container 3 and the supply tube 4.

Since the body fluid leakage-preventing agent 2 is pushed out from the syringe 10 by the piston 11, which slides inside the syringe 10, the body fluid leakage-preventing agent 2 can be reliably held in the syringe 10 before use, and the body fluid leakage-preventing agent 2 can be injected into the throat B by a simple operation of pushing the piston 11 into the syringe 10 during the injection operation.

Note that when disposing of the embalming device 1 after the body fluid leakage-preventing agent 2 is injected into the throat B, the container 3 and the supply tube 4 may be connected together or may be separated from each other. In order to separate the container 3 and the supply tube 4 from each other, the nozzle portion 13 of the syringe 10 may be pulled hard off the connecting portion 20 of the supply tube 4.

Although not shown, a protruding portion as an engagement portion may be provided on the inner periphery surface of the connecting portion 20 of the supply tube 4 while a groove portion as an engagement portion may be provided on the outer periphery surface of the nozzle portion 13, whereby they can be engaged with each other.

Through holes (engagement portions) 30, into which the protruding portions 14 of the nozzle portion 13 are fit, may be provided in the peripheral wall portion of the connecting portion 20 of the supply tube 4, as in a variation of Embodiment 1 shown in FIG. 8. In this variation, the through hole 30 and the protruding portions 14 can be engaged with each other by fitting the protruding portions 14 of the nozzle portion 13 into the through holes 30. Although not shown, a through hole (an engagement portion) may be provided in the peripheral wall portion of the nozzle portion 13, and a protruding portion (an engagement portion) to be fit into the through hole may be provided on the inner periphery surface of the connecting portion 20.

### Second Embodiment

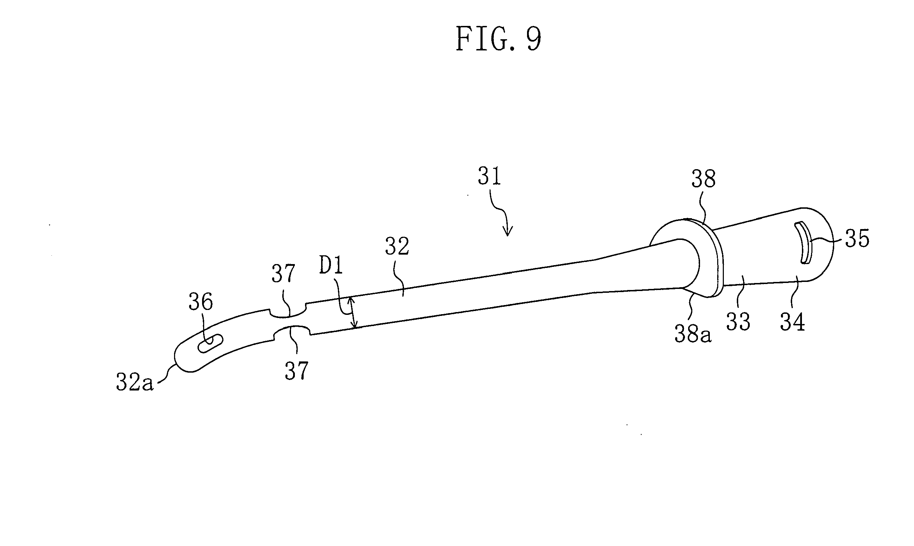

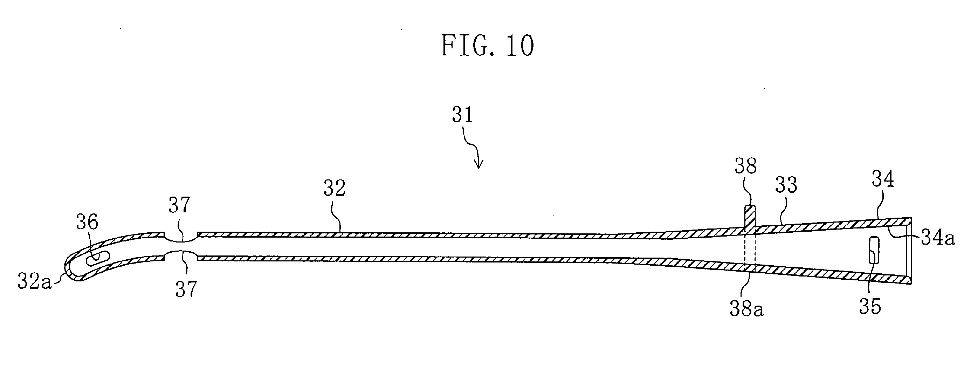

FIGS. 9 and 10 show a supply tube 31 used in a connection structure between a container and a supply tube according to Embodiment 2 of the present invention.

A predetermined area on the distal end side in the insertion direction of an inserted tube portion 32 of the supply tube 31 of Embodiment 2 is curved so that the distal end portion thereof is located toward one side in the radial direction with respect to the proximal end portion thereof. The first injection hole 16 is not opened in the distal end surface of the inserted tube portion 32, but the inserted tube portion 32 is closed by a curved surface portion 32a, which is curved so as to bulge in the insertion direction of the inserted tube portion 32. As with the second and third injection holes 17, 17, 18 and 18 of Embodiment 1 described above, a total of four injection holes 36, 36, 37 and 37 are formed in the peripheral wall portion of the distal end side of the inserted tube portion 32.

The outer diameter of the proximal end side of the inserted tube portion 32 is gradually increasing toward the proximal end. The thickness of the proximal end side of the inserted tube portion 32 is greater than that of the distal end side. An intermediate portion 33 that connects to the proximal end portion of the inserted tube portion 32 and a connecting portion 34 that connects to the intermediate portion 33 have generally the same thickness. Therefore, the outer periphery surface of the intermediate portion 33 and the outer periphery surface of the connecting portion 34 smoothly connect to each other, and the inner periphery surface of the intermediate portion 33 and the inner periphery surface of the connecting portion 34 also smoothly connect to each other.

The outer periphery surface of the connecting portion 34 is formed by an engagement surface 34a, as in Embodiment 1. Two through holes (engagement portions) 35 and 35, apart from each other in the circumferential direction, are formed in the peripheral wall portion of the connecting portion 34. Each through hole 35 has a shape that extends in the circumferential direction of the connecting portion 34. The through holes 35 are formed by punching dies provided at positions corresponding to the through holes 35 when molding the supply tube 31 by using an injection mold (not shown). Thus, it is possible to form the through holes 35 at a lower cost than when the through holes 35 are formed through a post-molding process.

As is the stopper 22 of Embodiment 1, a stopper 38 for preventing the over-insertion of the inserted tube portion 32 is formed on the outer periphery surface of the supply tube 31. A portion of the stopper 38 in the circumferential direction is cut off in a straight line, and the cut-off portion forms a straight portion 38a. The position of the straight portion 38a in the circumferential direction corresponds to the direction in which the distal end side of the inserted tube portion 32 is curved. Thus, as the position of the straight portion 38a in the circumferential direction corresponds to the direction in which the inserted tube portion 32 is curved, the operator can check, from outside the nasal cavity A, the direction in which the inserted tube portion 32 is curved with the inserted tube portion 32 inserted in the throat B.

Note that since the container used in the connection structure of Embodiment 2 is the same as the container 3 of Embodiment 1, like reference numerals to those of Embodiment 1 will be used in the following description.

In Embodiment 2, since the through holes 35 and 35 are formed in the connecting portion 34 of the supply tube 31, the operator, when connecting the container 3 and the supply tube 31 together, can visually check, from outside the connecting portion 34, whether the protruding portions 14 of the nozzle portion 13 of the container 3 are fit in and engaged with the through holes 35.

Also in Embodiment 2, as in Embodiment 1, by engaging the protruding portions 14 of the nozzle portion 13 with the through holes 35 of the connecting portion 34, it is possible to ensure the connection between the container 3 and the supply tube 31, thereby preventing the body fluid leakage-preventing agent 2 from leaking out from the connection portion and preventing the supply tube 31 from coming off the container 3.

When inserting the supply tube 31 of Embodiment 2 toward the throat B, the inserted tube portion 32 can be inserted smoothly into the throat B since the inserted tube portion 32 is curved so as to conform with the shape of the area from the nasal cavity A to the throat B. Note that if the inserted tube portion 32 is curved to an excessive degree, the operation of inserting it into the nasal cavity A will become difficult. Therefore, for example, the inserted tube portion 32 is preferably curved so that the center of the distal end portion of the inserted tube portion 32 is radially displaced from the center of the proximal end portion thereof by an amount in the range from one half to three times the diameter of the distal end of the inserted tube portion 32.

Moreover, since the straight portion 38a is formed by cutting off a portion of the stopper 38 so that the position of the straight portion 38a corresponds to the direction in which the inserted tube portion 32 is curved, the stopper 38, when coming close to the nose tip A1, is less likely to bump against the skin between the nose tip A1 and the upper lip (the area under the nose), thus improving the insertion operability.

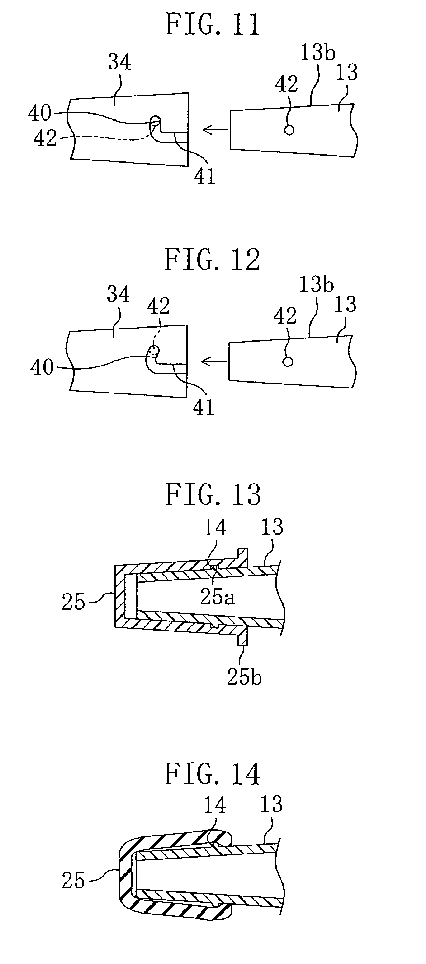

As in Variation 1 shown in FIG. 11, a first groove portion 40 and a second groove portion 41, as engagement portions, may be formed on the peripheral wall portion of the connecting portion 34 of the supply tube 31, and a pin portion (an engagement portion) 42 to be inserted into the first groove portion 40 and the second groove portion 41 may be formed on the peripheral wall portion of the nozzle portion 13 of the container 3. The first groove portion 40 extends in the circumferential direction in an axially intermediate portion of the connecting portion 34. The second groove portion 41 continuously extends with one end portion of the first groove portion 40, and extends in the axial direction along the peripheral wall portion of the connecting portion 34, reaching the proximal end portion of the connecting portion 34.

In Variation 1, in order to connect the container 3 and the supply tube 31 together, the nozzle portion 13 is first inserted into the connecting portion 34, and the pin portion 42 is inserted into the second groove portion 41. When the pin portion 42 reaches the distal end side of the second groove portion 41, the connecting portion 34 is rotated with respect to the nozzle portion 13 so as to position the pin portion 42 in the first groove portion 40, and then the pin portion 42 is positioned in an end portion of the first groove portion 40 that is opposite to the second groove portion 41, as shown in a phantom line. Thus, the pin portion 42 and the first groove portion 40 are engaged with each other, thereby preventing the nozzle portion 13 from coming off. By rotating the connecting portion 34 with respect to the nozzle portion 13, the tapered surface portion 13b of the nozzle portion 13 and the engagement surface 34a of the connecting portion 34 can be brought strongly into close contact with each other. Note that when connecting the container 3 and the supply tube 31 together, the nozzle portion 13 can be rotated with respect to the connecting portion 34.

The first groove portion 40 of Variation 1 may be formed so that a portion thereof further away from the second groove portion 41 is positioned closer to the proximal end side of the connecting portion 34, as in Variation 2 shown in FIG. 12. Then, the relative rotation between the connecting portion 34 and the nozzle portion 13 is suppressed when the pin portion 42 is in the first groove portion 40, thereby making it less likely that the pin portion 42 comes off the first and second groove portions 40 and 41. The first groove portion 40 may be formed so that a portion thereof further away from the second groove portion 41 is positioned closer to the distal end side of the connecting portion 34.

Note that although not shown, a first groove portion and a second groove portion may be formed in the nozzle portion 13, and a pin portion may be formed on the inner periphery surface of the connecting portion 34. In Variations 1 and 2, instead of the first groove portion 40 and the second groove portion 41, there may be formed a first depressed portion and a second depressed portion extending similarly to the first groove portion 40 and the second groove portion 41.

An engagement portion 25a, which is a depressed portion, may be provided on the cap 25, so that the engagement portion 25a is fit over the protruding portions 14 of the nozzle portion 13, as in Variation 3 shown in FIG. 13. The cap 25 has a cylindrical shape with the bottom, and the engagement portion 25a is formed so as to extend in the circumferential direction on the inner periphery surface of the cap 25. The engagement portion 25a is not limited to a depressed portion, but may be a through hole, for example.

A flange portion 25b is provided in the end portion of the cap 25 on the open side thereof. When the cap 25 is taken off the nozzle portion 13, it is easily taken off by putting fingers on the flange portion 25b. The shape of the flange portion 25b may be a shape continuously extending in the circumferential direction while protruding from the outer periphery surface of the cap 25, or may be a shape protruding only in a portion in the circumferential direction. Although not shown, a ring-shaped portion on which fingers can be placed may be provided on the cap 25.

The cap 25 may be made of a rubber, as in Variation 4 shown in FIG. 14. The cap 25 is formed so that the diameter on the open side thereof is smaller than the outer diameter of the nozzle portion 13. Therefore, when the cap 25 is on the nozzle portion 13, the cap 25 hangs on the protruding portions 14 of the nozzle portion 13 and is less likely to come off.

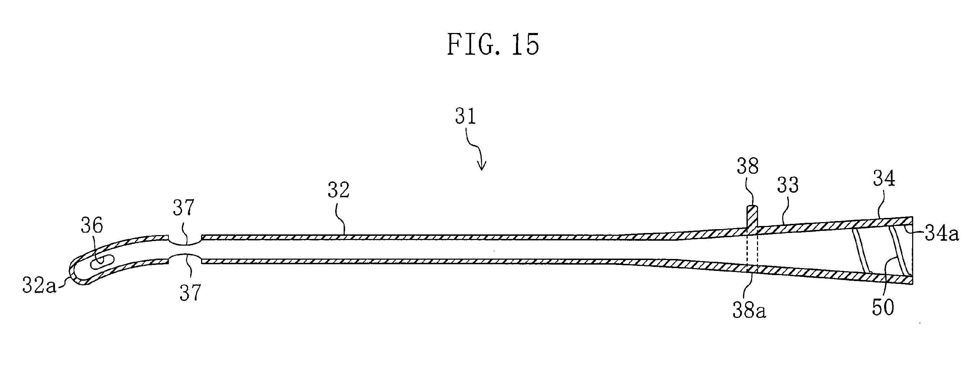

The engagement portion of the connecting portion 34 of the supply tube 31 may be formed by an internal thread portion 50, as in Variation 5 shown in FIG. 15. The internal thread portion 50 is formed in a spiral pattern on the engagement surface 34a of the connecting portion 34. In Variation 5, the engagement portion of the nozzle portion 13 is formed by an external thread portion (an engagement portion) that screws with the internal thread portion 50, although not shown. The external thread portion and the internal thread portion 50 are engaged together by being screwed together.

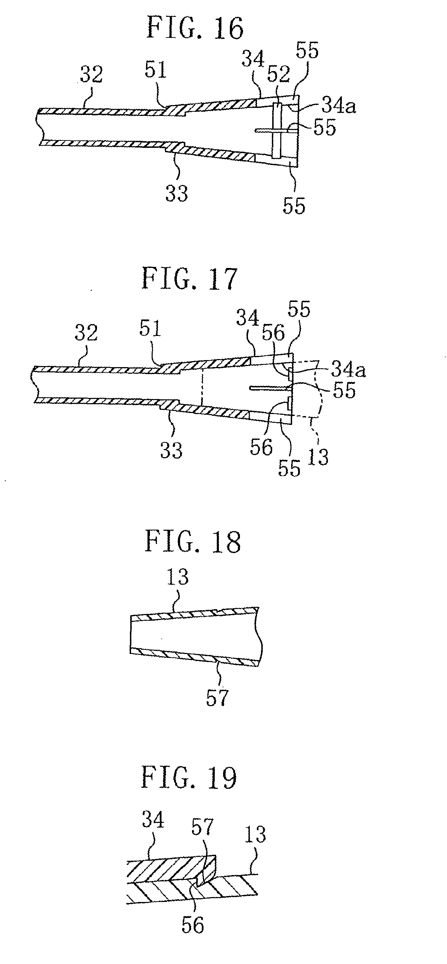

The outer diameter of the intermediate portion 33 of the supply tube 31 may be made larger than the outer diameter of the inserted tube portion 32 to thereby form a step portion 51 on the outer periphery surface of the supply tube 31, as in Variation 6 shown in FIG. 16. The step portion 51 forms a stopper that abuts on the nose tip A 1 when the first to third injection holes 16 to 18 of the inserted tube portion 32 reach the throat B or the vicinity of the throat B.

In Variation 6, four slits 55, 55, ..., extending in the axial direction while being spaced apart from one another in the circumferential direction, are formed on the connecting portion 34 of the supply tube 31. A groove portion 52 as an engagement portion is formed on the engagement surface 34a of the connecting portion 34. With the slits 55, 55, ..., formed in the connecting portion 34, the connecting portion 34 deforms when the nozzle portion 13 is inserted into the connecting portion 34, so that the protruding portions 14 of the nozzle portion 13 are more easily fit into the groove portion 52 of the connecting portion 34.

The engagement portion of the connecting portion 34 of the supply tube 31 may be formed by claw portions 56, and the engagement portion of the nozzle portion 13 may be a depressed portion 57 into which the claw portions 56 are fit, as in Variation 7 shown in FIGS. 17 to 19. The step portion 51 serving as a stopper is formed on the supply tube 31 of Variation 7, as in Variation 6. The slits 55, 55, ..., are formed in the connecting portion 34, as in Variation 6. Four of the claw portions 56 are provided spaced apart from one another in the circumferential direction of the connecting portion 34, and protruding from the proximal end portion of the connecting portion 34 toward the inside of the connecting portion 34. On the other hand, the depressed portion 57 continuously extends in the circumferential direction of the nozzle portion 13.

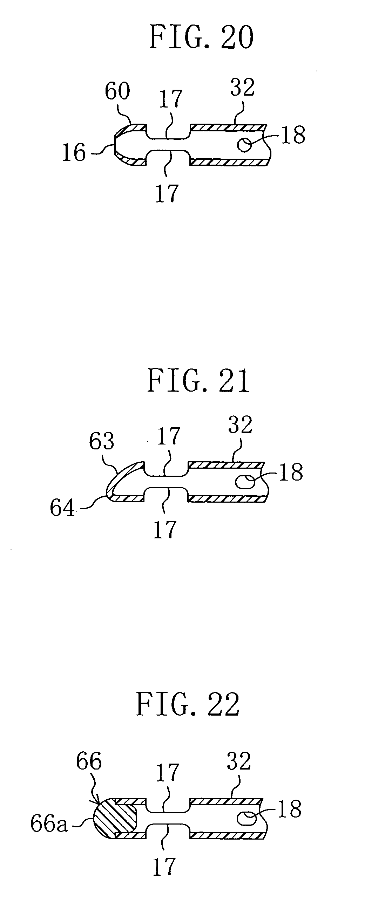

A tapered portion 60 whose diameter decreases toward the distal end may be provided on the distal end side of the inserted tube portion 32 of the supply tube 31, as in Variation 8 shown in FIG. 20. The first injection hole 16, which is a small aperture, is opened in the distal end surface of the tapered portion 60. The third injection holes 18 are in a circular shape. In Variation 8, if the second and third injection holes 17 and 18 are closed by the inner wall of the nasal cavity A, or the like, the body fluid leakage-preventing agent 2 can be injected into the throat B from the first injection hole 16. With the first injection hole 16 opened in the tapered portion 60, the rigidity of the tapered portion 60 is decreased, and the tapered portion 60 can be easily bent. Thus, when inserting the inserted tube portion 32 toward the throat B, the tapered portion 60 can deform and can be smoothly inserted deep into the nasal cavity A.

The distal end surface of the inserted tube portion 32 of the supply tube 31 may be closed by a curved surface portion 63, without forming the first injection hole therein, as in Variation 9 shown in FIG. 21. A protruding portion 64 is formed in the curved surface portion 63, and the protruding portion 64 is radially shifted from the axial line of the inserted tube portion 32. The second injection holes 17 and 17 are formed both on the upper and lower sides of the inserted tube portion 32 shown in FIG. 21. With the second injection holes 17 and 17, the distal end side of the inserted tube portion 32 is more easily bent, and the curved surface portion 63 can be easily displaced in the vertical direction of FIG. 21 (the radial direction of the inserted tube portion 32). If the protruding portion 64 abuts on the inner wall of the nasal cavity A when inserting the inserted tube portion 32, the distal end side of the inserted tube portion 32 is easily bent toward the throat B, thus enabling smooth insertion of the inserted tube portion 32.

A lid member 66 may be fit into the distal end portion of the inserted tube portion 32 of the supply tube 31, as in Variation 10 shown in FIG. 22. The outer surface of the lid member 66 is formed by a curved surface 66a, which is curved toward the insertion direction of the inserted tube portion 32. The shape of the curved surface 66a of the lid member 66 may be the same as that of the distal end surface of the inserted tube portion 32 of Variation 9.

The first to third injection holes 16 to 18 may each be formed by making a cross-shaped cut in the inserted tube portion 32. Then, when injecting the body fluid leakage-preventing agent 2, the cut is opened wide by the discharging pressure, thereby allowing the body fluid leakage-preventing agent 2 to be discharged therethrough. Since the cut is closed after the injection, the body fluid leakage-preventing agent 2 in the inserted tube portion 32 is less likely to leak out when the inserted tube portion 32 is pulled out of the nasal cavity A. The shape of the cut may be straight or curved.

The shape of the first to third injection holes 16 to 18 is not limited to a circular shape or an elliptical shape, but may be, for example, a straight slit shape, a bow-shaped curved slit shape, a polygonal shape, or the like.

Three each of the second and third injection holes 17 and 18 may be opened at a 120° interval in the circumferential direction of the inserted tube portion 32, or four each of them may be opened at a 90° interval.

One of the first to third injection holes 16 to 18 may be formed by a cut.

Separately from the second injection holes 17, there may be formed injection holes in the inserted tube portion 32, although not shown, that are axially away from the third injection holes 18.

Although not shown, a one-way valve formed by a thin film material may be provided on the outer side of the first to third injection holes 16 to 18, for example, thereby suppressing the dripping of the body fluid leakage-preventing agent 2, left in the inserted tube portion 32, through the injection holes 16 to 18 after use. A portion of the thin film material may be attached to the outer periphery surface of the inserted tube portion 32 so that when the body fluid leakage-preventing agent 2 in the inserted tube portion 32 is injected, the thin film material comes off the outer periphery surface, except for the attached portion, due to the discharging pressure. Thus, during the injection, the first to third injection holes 16 to 18 are opened by the discharging pressure of the body fluid leakage-preventing agent 2, and the body fluid leakage-preventing agent 2 is injected into the throat B. Then, after the injection, the piston 11 is slightly pulled back so as to give a negative pressure inside the storage section 12, thereby sucking the body fluid leakage-preventing agent 2 in the inserted tube portion 32 into the syringe 10. This brings the thin film material into close contact with the outer periphery surface of the inserted tube portion 32, thereby closing the first to third injection holes 16 to 18. Any one or two of the first to third injection holes 16 to 18 may be closed by the thin film materials.

Variations 1 to 10 of Embodiment 2 are also applicable to Embodiment 1.

The protruding portions 14 of the nozzle portion 13 of the container 3, or the distal end side thereof with respect to the protruding portions 14, may be colored so that it can be clearly distinguished from other portions, and the connecting portion 34 of the supply tube 31 may be formed by a light-transmitting material. Thus, the protruding portions 14 of the nozzle portion 13 can be seen through from the outside of the connecting portion 34, thereby enabling one to visually check whether the protruding portions 14 are engaged with the through holes 35.

### Third Embodiment

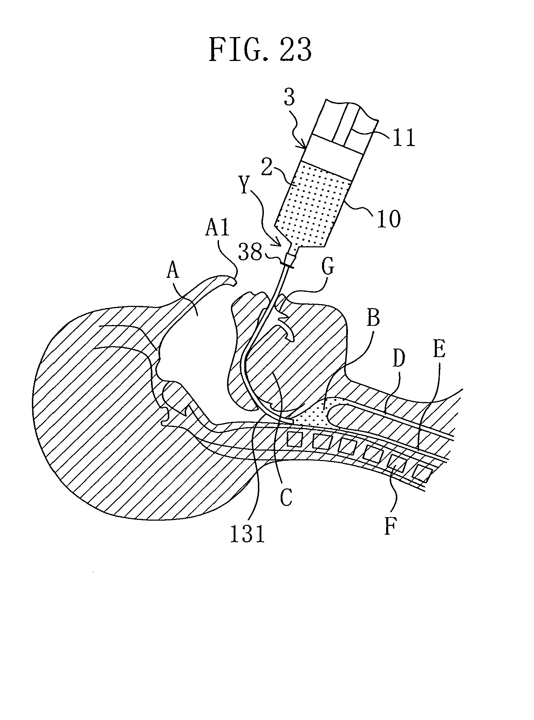

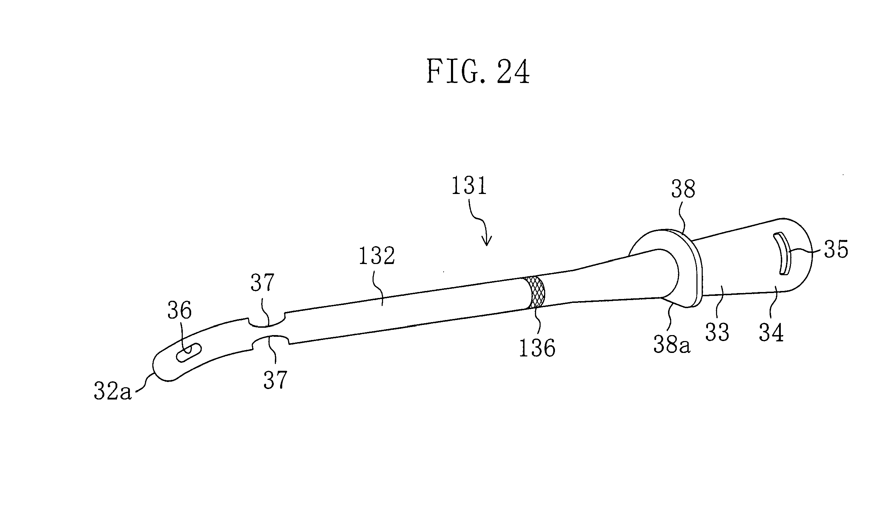

Referring to FIGS. 23 and 24, a connection structure between a container and a supply tube according to Embodiment 3 of the present invention will now be described.

With the structures of Embodiments 1 and 2, the supply tube 4 is inserted through the nasal cavity A into the throat B. Embodiment 3 differs from Embodiments 1 and 2 in that a supply tube 131 is inserted through the oral cavity G into the throat B, as shown in FIG. 23. Note that the supply tube 4 of Embodiment 3 may be inserted through the nasal cavity A into the throat B. In such a case, the supply tube 131 serves as a multipurpose supply tube that can be used both when it is inserted through the nasal cavity A and when it is inserted through the oral cavity G.

The supply tube 131 of Embodiment 3 differs, structure-wise, from the supply tube 31 of Embodiment 2 in that a marking 136 is provided on an inserted tube portion 132, with the length and the thickness thereof being equal to those of Embodiment 2. The marking 136 is located away from the stopper 38 toward the distal end side of the inserted tube portion 132. The marking 136 is formed on the outer periphery surface of the inserted tube portion 132 in a band shape with a predetermined width extending in the circumferential direction, and is colored with a different color from the color of the inserted tube portion 132. The marking 136 may be of a single color or multiple colors, or may be a pattern, letters, or the like.

The marking 136 is used when it is inserted from the oral cavity G to the throat B. That is, the distance from the mouth to the throat B through the oral cavity G is typically shorter than the distance from the nose tip A1 to the throat B through the nasal cavity A. Therefore, when the inserted tube portion 132 is inserted from the mouth to the throat B, if it is inserted until the stopper 38 whose position is determined for the insertion through the nasal cavity A abuts on the lips or the front teeth, for example, the injection ports 36 and 37 will be inserted deeper than the appropriate position in the throat B, and the body fluid leakage-preventing agent 2 may not be injected into the intended area of the throat B.

That is, in the operation of inserting the inserted tube portion 132, the over-insertion of the inserted tube portion 132 can be prevented by stopping the insertion when the marking 136 comes close to the lips or the front teeth before the stopper 38 abuts on the lips or the front teeth, as shown in FIG. 23. Then, it is possible to reliably inject the body fluid leakage-preventing agent 2 to the intended position of the throat B.

When the supply tube 131 is inserted from the nasal cavity A to the throat B, it can be inserted until the stopper 38 abuts on the nose tip A1 of the nasal cavity A. Then, it is possible to reliably inject the body fluid leakage-preventing agent 2 to the intended position of the throat B. The stopper 38 can be used also as a marking indicating the amount of insertion of the inserted tube portion 132 into the throat B.

Embodiment 3 can provide similar effects to those of Embodiment 1.

Note that the marking 136 is not limited to the marking as described above, but may be, for example, a step formed on the outer periphery surface of the inserted tube portion 132, a portion of the outer periphery surface of the inserted tube portion 132 where the surface is roughened, or a portion of the outer periphery that is cut and raised so that it serves as a stopper. Although not shown, a separate stopper formed by a separate member from the inserted tube portion 132 may be provided on the inserted tube portion 132 so that the separate stopper can be moved along on the outer periphery surface of the inserted tube portion 132. Then, it is possible to easily accommodate the case where the inserted tube portion 132 is inserted into the throat B from the nasal cavity A and the case where the inserted tube portion 132 is inserted into the throat B from the oral cavity G by changing the position of the separate stopper. Thus, the separate stopper can be used in both cases. Note that the separate stopper can be used also as a marking indicating the amount of insertion of the inserted tube portion 132 into the throat B.

Although not shown, a ring-shaped stopper formed by a separate member may be provided on the inserted tube portion 132 closer to the distal end side thereof than the stopper 38. Then, by moving the ring-shaped stopper to a corresponding position depending on whether the inserted tube portion 132 is inserted into the throat B from the nasal cavity A or the inserted tube portion 132 is inserted into the throat B from the oral cavity G, the ring-shaped stopper can be used in both cases.

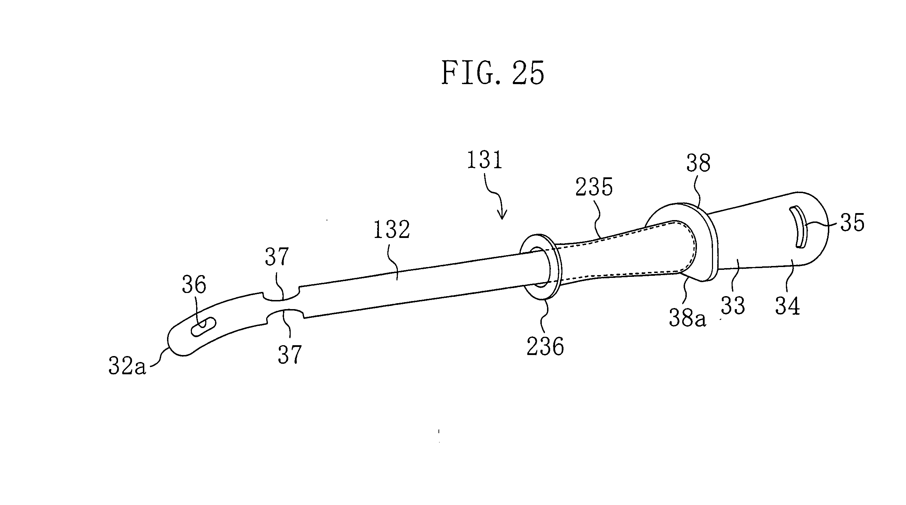

FIG. 25 shows Variation 1 of Embodiment 3. In Variation 1 of Embodiment 3, a cover tube 235 opened at both ends is put over the inserted tube portion 132. The cover tube 235 serves as one or both of the stopper and the marking described above, and can be attached to, or detached from, the supply tube 131. A flange portion 236 is formed in one axial end portion of the cover tube 235. The inner diameter of the cover tube 235 is set to be smaller than the outer diameter of the proximal end side of the inserted tube portion 132.

When using the structure of Variation 1, the cover tube 235 is set over the inserted tube portion 132 of the supply tube 131 before the inserted tube portion 132 is inserted into the oral cavity G. At this point, the cover tube 235 is oriented so that the opposite side of the cover tube 235 to the flange portion 236 abuts on the stopper 38. Then, as the cover tube 235 is moved until the opposite side of the cover tube 235 to the flange portion 236 abuts on the stopper 38, the inner periphery surface of the cover tube 235 is pressed against the outer periphery surface of the inserted tube portion 132 since the inner diameter of the cover tube 235 is set to be smaller than the outer diameter of the proximal end side of the inserted tube portion 132. Thus, the cover tube 235 does not easily come off the inserted tube portion 132. Note that the coming off of the cover tube 235 may be suppressed by providing a projecting portion on the inner periphery surface of the cover tube 235 so that the projecting portion abuts on the outer periphery surface of the inserted tube portion 132.

Thus, after the cover tube 235 is set over the inserted tube portion 132, the inserted tube portion 132 is inserted into the oral cavity G until the flange portion 236 of the cover tube 235 abuts on the lips or the front teeth. Then, it is possible to position the injection ports 36 and 37 at an appropriate place in the throat B. The cover tube 235 may be set at a position that is short of abutting on the stopper 38.

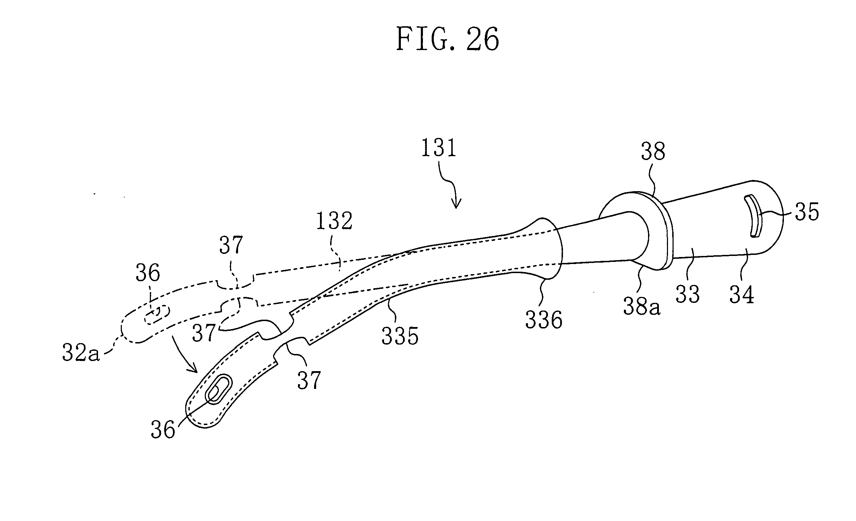

FIG. 26 shows Variation 2 of Embodiment 3. In Variation 2 of Embodiment 3, a cover tube 335 having the bottom is put over the inserted tube portion 32. The distal end portion of the cover tube 335 is formed by a curved surface. Openings that coincide with the injection ports 36 and 37 of the inserted tube portion 132 are formed on the distal end side of the cover tube 335. A flared portion 336 is formed on the proximal end side of the cover tube 335. The flared portion 336 serves as one or both of the stopper and the marking described above. The inner diameter of the cover tube 335 is set to be smaller than the outer diameter of the proximal end side of the inserted tube portion 132. Moreover, the cover tube 335 is curved as a whole, and the degree of the curve is set to be greater than the degree of the curve of the inserted tube portion 132 (shown in a phantom line in the figure).

When using the structure of Variation 2, the cover tube 335 is set over the inserted tube portion 132 of the supply tube 131 before the inserted tube portion 132 is inserted into the oral cavity G. At this point, the openings on the distal end side of the cover tube 335 are aligned with the injection ports 36 and 37 of the inserted tube portion 132. Then, the distal end portion of the inserted tube portion 132 is brought into contact with the inner surface of the distal end portion of the cover tube 335, thus positioning the cover tube 335.

After the cover tube 335 is set over the inserted tube portion 132 as described above, the inserted tube portion 132, together with the cover tube 335, is inserted into the oral cavity G until the flared portion 336 of the cover tube 235 abuts on the lips or the front teeth. Thus, the injection ports 36 and 37 of the inserted tube portion 132 can be positioned at an appropriate position in the throat B.

In Variation 2, since the cover tube 335 is put over the inserted tube portion 132, the outer diameter of the portion to be inserted into the throat B is increased. Nevertheless, this will present no problems during the insertion into the throat B since the oral cavity G can be opened wider than the nasal cavity A. By putting the cover tube 335 over the inserted tube portion 132, the inserted tube portion 132 is reinforced and less easily bent. Thus, when inserting the inserted tube portion 132 into the oral cavity G, the inserted tube portion 132 will not bend unnecessarily, enabling smooth insertion. With the increased diameter, the operator can know, from the external appearance, that the tube is one that is inserted into the throat B through the oral cavity G.

Note that instead of the cover tubes 235 and 335 described above, a stopper and/or a marking may be provided by a member formed so that it can be fit over the inserted tube portion 132, for example. An example of such a member is a bellows-shaped tube member, but it is not limited thereto. A tape may be wound around the inserted tube portion 132, which may be used as a stopper and/or a marking. An elastic resin ring member having a C-shaped cross section may be fit around the inserted tube portion 132, which may be used as a stopper and/or a marking. When the elastic ring member is attached to the inserted tube portion 132, the elastic ring member can be radially expanded, and the operation of attaching it to the inserted tube portion 132 is easy.

### Fourth Embodiment

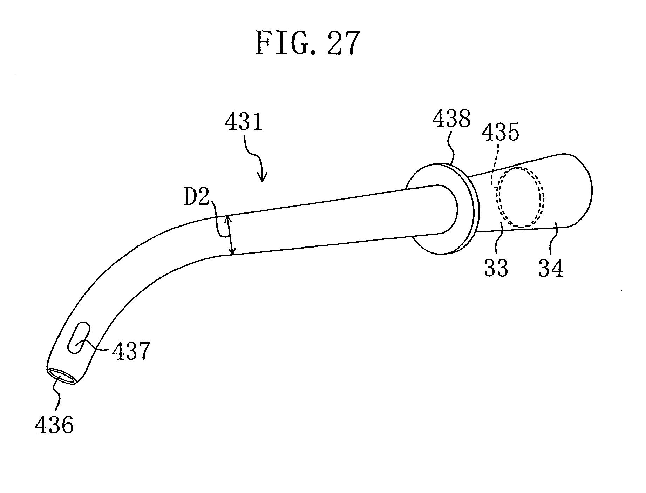

FIG. 27 shows a supply tube 431 according to Embodiment 4 of the present invention. The supply tube 431 is used only for the insertion into the throat B through the oral cavity G, and the length thereof is set to be shorter than those of Embodiments 1 to 3. The outer diameter D2 of the supply tube 431 of Embodiment 4 is set to be greater than the outer diameter D1 of Embodiment 2 (shown in FIG. 9).

An injection port 436 is formed in the distal end surface of the supply tube 431. Two injection ports 437 (only one is shown in FIG. 27), spaced apart from each other in the circumferential direction, are provided in the peripheral wall portion of the distal end side of the supply tube 431. A stopper 438 having a similar function to that of Embodiment 1 is provided on the supply tube 431. The stopper 438 is in a disc shape. The supply tube 431 is curved so as to conform with the curved shape of the oral cavity G. The supply tube 431 has a high rigidity so that it undergoes little deformation when inserted into the oral cavity G.

A case where the tube of Embodiment 4 is used will be described. As the supply tube 431 is inserted from the oral cavity G toward the throat B, the stopper 438 of the supply tube 431 abuts on the lips or the front teeth, thereby optimally positioning the injection ports 436 and 437 in the throat B. When inserting the supply tube 431, the mouth may be opened wide, if rigor mortis has not set in in the corpse. With the supply tube 431 inserted in the throat B, the supply tube 431 will not deform to decrease the inner diameter thereof, and the body fluid leakage-preventing agent 2 can be injected smoothly into the throat B, since the supply tube 431 has a high rigidity and is curved so as to conform with the shape of the oral cavity G, as described above.

Note that the supply tube 431 may be elastically deformable. In such a case, the supply tube 431 deforms in conformity with the curved shape of the oral cavity G of the corpse.

A groove portion (an engagement portion) 435, similar to the groove portion (an engagement portion) 23 of Embodiment 1, is provided in the connecting portion 34. A through hole similar to that of Embodiment 2 may be formed, instead of the groove portion 435.

Embodiment 4 can provide similar effects to those of Embodiment 1.

The supply tubes 4 and 31 for the nasal cavity of Embodiments 1 and 2, which are designed for the insertion into the throat B through the nasal cavity A, and the supply tubes 131 and 431 for the oral cavity of Embodiments 3 and 4, which are designed for the insertion into the throat B through the oral cavity G may be provided as a set.

In Embodiments 1 to 4, a common preservative, antiseptic, antimicrobial, sterilant, aromatic, disinfectant, or the like, or a mixture thereof, may be added to the body fluid leakage-preventing agent 2. For example, stabilized chlorine dioxide, formalin, an alcohol, a phenol, an oil tapped from a tree, or the like, may be added to the body fluid leakage-preventing agent 2.

While the storage section 12 of the container 3 is formed by the syringe 10 in Embodiments 1 to 4, it is not limited thereto, but may be formed by using a flexible tubelike material. In such a case, the body fluid leakage-preventing agent 2 can be squeezed out of the storage section.

The supply tube 4 of Embodiment 1 or the supply tube 31 of Embodiment 2 may be inserted toward the throat B from the oral cavity G (shown in FIG. 7).

The connecting portions 20 and 34 and the inserted tube portions 15, 32 and 132 of the supply tubes 4, 31, 131 and 431 of Embodiments 1 to 4 may be separately molded each by using a resin, and then welded together into an integral piece. Then, different resins may be used for the connecting portions 20 and 34 and for the inserted tube portions 15, 32 and 132, thus improving the freedom in designing the supply tubes 4 and 31.

The material of the supply tubes 4, 31, 131 and 431 is not limited to a flexible resin material as described above, but may be a hard resin material, a metal material, a fiber material, or the like, for example.

The cross section of the inserted tube portions 15, 32 and 132 of the supply tubes 4, 31, 131 and 431 may be an elliptical shape, instead of a circular shape.

If the supply tube 4 or 31 is used with an infant, a marking or a stopper is preferably provided at a position about 5 cm away from the distal end of the supply tube 4 or 31. For use with a child, a marking or a stopper is preferably provided at a position about 8 cm away from the distal end of the inserted tube portion 15. If the supply tube 4 or 31 is used both for the insertion into the throat B through the nasal cavity A and for the insertion into the throat B through the oral cavity G, the marking or the stopper described above may be provided at two different positions. A marking for use with a child may be formed with a large width in the axial direction of the supply tube 4 or 31, wherein the distal end side of the marking (the distal end side of the supply tube 4 or 31) is used as a marking for the insertion through the oral cavity G, and the rear end side thereof (the proximal end side of the supply tube 4 or 31) is used as a marking for the insertion through the nasal cavity A. A supply tube for use with an infant, a supply tube for use with a child, and a supply tube for use with an adult may be provided as a set so that one of the supply tubes can be selected as necessary. The supply tube for use with an infant, the supply tube for use with a child, and the supply tube for use with an adult may be provided with different identification marks, or the supply tubes may be colored differently. The size of the container 3 may be varied between the infant, child and adult applications. The ingredient and the amount of injection of the body fluid leakage-preventing agent 2 may be varied between the infant, child and adult applications.

### INDUSTRIAL APPLICABILITY

As described above, a connection structure between a container and a supply tube, a container, and a supply tube of the present invention are applicable to applications where a body fluid leakage-preventing agent is injected into the throat of a corpse, for example.

## Claims

1. A connection structure between a container and a supply tube, wherein the container stores a body fluid leakage-preventing agent for a corpse, and the supply tube supplies the body fluid leakage-preventing agent into a throat of the corpse, wherein
the container includes a storage section of which an inside is filled with the body fluid leakage-preventing agent, a nozzle portion provided in the storage section, and an engagement portion provided in the nozzle portion,
the supply tube includes an inserted tube portion to be inserted toward the throat of the corpse,
an injection hole is formed on a distal end side of the inserted tube portion in an insertion direction,
a connecting portion to be connected to the nozzle portion of the container is provided on a proximal end side of the inserted tube portion, and
an engagement portion to be engaged with an engagement portion of the nozzle portion is provided in the connecting portion.

2. The connection structure between a container and a supply tube of claim 1, wherein
the inserted tube portion and the connecting portion of the supply tube are molded together.

3. The connection structure between a container and a supply tube of claim 1, wherein
the inserted tube portion and the connecting portion of the supply tube are separately molded each by using a resin material, and then welded together into an integral piece.

4. The connection structure between a container and a supply tube of one of claims 1 to 3, wherein
an outer periphery surface of the nozzle portion of the container is formed by a tapered surface with its diameter decreasing toward a distal end of the nozzle portion,
the connecting portion of the supply tube has a cylindrical shape such as to surround the tapered surface of the nozzle portion, and
an inner periphery surface of the connecting portion is formed so as to conform with the tapered surface.

5. The connection structure between a container and a supply tube of claim 4, wherein
the engagement portion of the nozzle portion is provided on the tapered surface, and
the engagement portion of the connecting portion is provided on the inner periphery surface of the connecting portion.

6. The connection structure between a container and a supply tube of claim 4 or 5, wherein
one of the engagement portion of the nozzle portion and the engagement portion of the connecting portion is a projecting portion, and the other is a depressed portion that fits around the projecting portion.

7. The connection structure between a container and a supply tube of claim 4 or 5, wherein
a through hole is formed in a peripheral wall portion of one of the nozzle portion and the connecting portion, and a protruding portion to be inserted into the through hole is formed on a peripheral wall portion of the other, the through hole and the protruding portion each being the engagement portion.

8. The connection structure between a container and a supply tube of claim 6, wherein
the depressed portion is a groove portion, and the projecting portion is a protruding portion to be inserted into the groove portion.

9. The connection structure between a container and a supply tube of claim 4 or 5, wherein
a peripheral wall portion of one of the nozzle portion and the connecting portion is provided with a first groove portion extending in a circumferential direction in an axially intermediate portion thereof, and a second groove portion continuously extending with the first groove portion and extending in an axial direction along the peripheral wall portion, and a peripheral wall portion of the other is provided with a pin portion to be inserted in the first groove portion and the second groove portion.

10. The connection structure between a container and a supply tube of claim 4 or 5, wherein
a peripheral wall portion of one of the nozzle portion and the connecting portion is provided with a first depressed portion extending in a circumferential direction in an axially intermediate portion thereof, and a second depressed portion continuously extending with the first depressed portion and extending in an axial direction along the peripheral wall portion, and a peripheral wall portion of the other is provided with a pin portion to be inserted in the first depressed portion and the second depressed portion.

11. The connection structure between a container and a supply tube of one of claims 1 to 10, wherein
a cap for closing a distal end opening of the nozzle portion of the container is engaged with the engagement portion of the nozzle portion.

12. The connection structure between a container and a supply tube of one of claims 1 to 11, wherein
the storage section of the container is a cylindrical portion of a syringe, and
a piston for pushing out the body fluid leakage-preventing agent is inserted into the cylindrical portion of the syringe.

13. The connection structure between a container and a supply tube of one of claims 1 to 12, wherein
the distal end side of the inserted tube portion of the supply tube is curved.

14. The connection structure between a container and a supply tube of one of claims 1 to 13, wherein
a tapered portion whose diameter decreases toward a distal end may be provided on the distal end side of the inserted tube portion of the supply tube.

15. The connection structure between a container and a supply tube of one of claims 1 to 14, wherein
a thickness of the inserted tube portion of the supply tube is set to be smaller than a thickness of the connecting portion.

16. The connection structure between a container and a supply tube of one of claims 1 to 15, wherein
the supply tube is provided with a mark indicating an amount of insertion of the inserted tube portion into the throat.

17. The connection structure between a container and a supply tube of one of claims 1 to 15, wherein
the supply tube is provided with a stopper that abuts on a portion of the corpse when the injection hole of the inserted tube portion inserted toward the throat reaches a predetermined position.

18. The connection structure between a container and a supply tube of one of claims 1 to 15, wherein
the supply tube is provided with a mark indicating an amount of insertion and/or a stopper for controlling the amount of insertion to be a predetermined amount when the inserted tube portion is inserted from a nasal cavity toward the throat, and a mark indicating an amount of insertion and/or a stopper for controlling the amount of insertion to be a predetermined amount when the inserted tube portion is inserted from an oral cavity toward the throat.

19. The connection structure between a container and a supply tube of one of claims 1 to 15, wherein
the supply tube is provided with a marking member indicating an amount of insertion of the inserted tube portion into the throat so that the marking member can be attached to, or detached from, the supply tube.

20. The connection structure between a container and a supply tube of one of claims 1 to 15, comprising:
a supply tube for a nasal cavity to be inserted from a nasal cavity toward the throat; and
a supply tube for an oral cavity to be inserted from an oral cavity toward the throat.

21. The connection structure between a container and a supply tube of one of claims 1 to 20, wherein
a plurality of the injection holes of the supply tube are formed in a peripheral wall portion of the inserted tube portion on the distal end side thereof.

22. A container to which a supply tube for supplying a body fluid leakage-preventing agent into a throat of a corpse is connected, the container comprising:
a storage section filled with the body fluid leakage-preventing agent; a nozzle portion provided in the storage section; and an engagement portion provided in the nozzle portion, the nozzle portion being connected to a connecting portion provided in the supply tube, wherein
the engagement portion of the nozzle portion is engaged with an engagement portion of the connecting portion.

23. A supply tube connected to a container storing a body fluid leakage-preventing agent for a corpse, comprising:
an inserted tube portion to be inserted toward a throat of the corpse, wherein
an injection hole is formed on a distal end side of the inserted tube portion in an insertion direction,
a connecting portion to be connected to a nozzle portion provided in the container is provided on a proximal end side of the inserted tube portion, and
the connecting portion is provided with an engagement portion to be engaged with an engagement portion of the nozzle portion.
